# EUROPEAN PATENT APPLICATION

(11) **EP 2 363 388 A1**
(43) Date of publication of application: **07.09.2011**
(21) Application number: 10155238.8
(22) Date of filing: 02.03.2010
(51) Int. Cl.: C07C 209/62, C07C 231/18, B01J 31/24, C07F 15/00, C07F 17/02, C07C 211/29, C07C 233/13, C07B 53/00

(54) **Process for the production of chiral amines**

(71) Applicant: DSM IP Assets B.V., 6411 TE Heerlen (NL)
(72) Inventor: The designation of the inventor has not yet been filed
(74) Representative: Duffy, James Edwin Marsh

(57) **Abstract**

The invention relates to a process for the preparation of optically active N-protected secondary amines by asymmetric hydrogenation in the presence of an optically active transition metal catalyst, one or more ligands, a nitrogen protecting reagent and optionally an additive, wherein the substrate is an imine.

## Description

The invention relates to a process for the asymmetric hydrogenation of imines, using a transition metal catalyst and a ligand and to the N-protected amines obtained therewith.

Methods to produce optically active amines by asymmetric hydrogenation of the corresponding imines using chiral transition metal complexes have been described before (e.g. Blaser and Spindler, Organic Reactions, Vol. 74, Chapter 1, Ed. Denmark et al., Wiley, 2009).

Despite the efforts and the potential, the asymmetric hydrogenation of imines is still a slowly developing field. This is caused by the fact that all systems, especially when reactions are performed at scale, show catalyst deactivation, the extent of which depends on catalyst precursor, ligand, substrate used and product obtained, preventing the achievements of industrially acceptable protocols (e.g. Spindler and Blaser, Handbook of Homogeneous Catalysis, Vol. 3, Chapter 34, Ed. de Vries and Elsevier, Wiley, 2007).

It has been demonstrated that additives help fighting catalyst deactivation. For example, in the iridium-catalyzed asymmetric hydrogenation of imines the addition of I₂, ammonium halides and acids has shown to prevent clusters formation which causes catalyst deactivation (e.g. Cha and Osborn J. Am. Chem. Soc. 1990, 112, 9400). In this respect, a process was disclosed by Ciba-Geigy (US6822118 to Jalett et al.), in which, by using such additives, high activities were obtained. In the best examples reported, high temperatures and hydrogen pressures have been used (80°C and 80 bar H₂).

Recently, Zhou et al. (J. Am. Chem. Soc. 2010, 128, 12886) have reported the asymmetric hydrogenation of N-aryl imines at low hydrogen pressure (1-20 bar), but in order to obtain good conversions high catalyst loading was used (1 mol%). A limited amount of prior art deals with the metal-catalyzed asymmetric hydrogenation of imines yielding N-alkyl secondary amines, for which catalyst productivity showed to be far from optimal, as disclosed by Pfaltz et al. (Chem. Eur. J. 1997, 3, 887) using an iridium-based catalyst and Morris et al. (Organomet. 2001, 20, 1047) using a ruthenium-based catalyst. Poor activities have been also recently reported by Zhang et al. (J. Am. Chem. Soc. 2010, 132, 2124), whilst disclosing the asymmetric hydrogenation of imine salts toward primary amines catalyzed by iridium-based complexes.

Clearly, the major drawback of the asymmetric hydrogenation of imines disclosed in the prior art is that these reductions often result in low activities and use of reaction conditions which are prohibitive for industrial scale processes, such as high catalyst loading, high hydrogen pressure and high temperature.

Surprisingly, it has now been found that catalyst activity can be substantially increased by preventing product inhibition, caused by the weak complexation of the amine hydrogenation product to the metal center, by the in situ protection of its nitrogen group. This allows using low catalyst loadings, low hydrogen pressures at room temperature.

The invention now relates to a process for the asymmetric hydrogenation of imines according to formula (I) 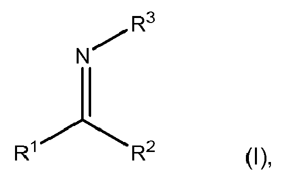 and salts thereof, wherein R¹ and R² are each independently any alkyl, aryl, aralkyl, alkenyl or alkynyl group with 1-20 C-atoms and R³ is hydrogen atom, an alkyl, aralkyl, alkenyl or alkynyl group with 1-20 C-atoms and wherein R¹, R² and R³ can optionally form a ring together, it being possible for R¹, R² and R³ to also contain one or more heteroatoms and substituents, in the presence of a transition metal catalyst, one or more ligands, a nitrogen protecting reagent and optionally an additive, wherein a product of formula (II) and salts thereof 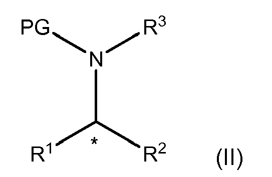 are formed, wherein PG stands for a protecting group introduced by the reaction of the nitrogen protecting reagent with the product of the asymmetric hydrogenation reaction.

Optionally R¹, R² and R³ can form a ring together giving rise to cycloalkyl, heterocyclic alkyl, aromatic and heteroaromatic rings of 3-8 C-atoms.

Preferably R¹ and R² each independently represent an aryl, linear or branched alkyl, aralkyl, alkenyl or alkynyl group with 1-20 C-atoms which might or might not contain additional heteroatoms and/or functional groups. Suitable additional heteroatoms are one or more from the group of O, S, N and P. Suitable substituents are functional groups that can be chosen, for example, among C₁-C₆ haloalkyl, -F, -Cl, - Br, -CN, -NO₂, -CO₂H, -CONH₂, -SO₃H, -PO₃H₂ or C₁-C₁₂ alkyl esters or amides, phenyl esters or benzyl esters of the groups -CO₂H -CONH₂, -SO₃H or -PO₃H₂.

More preferably, either R¹ or R² represent an aryl group. Suitable substituents for the aryl groups include, but are not limited to one or more hydrogen, aryl, alkyl, alkoxy (-OR⁴), carboxylate (-CO(O)R⁴), carbonyl (-C(O)R⁴), nitro (-NO₂), cyano (-CN), amino (-NR⁴R⁵) or methylene halide groups, wherein R⁴ and R⁵ each independently represent a hydrogen, aryl, linear or branched alkyl, aralkyl or alkenyl group with 1-12 C-atoms which might or might not contain additional heteroatoms and/or functional groups; wherein the carbonyl groups also include but are not limited to ketones, aldehydes and amides, and wherein the methylene halide groups are of the formula ―CXₙH₃₋ₙ, wherein X can be fluoride, chlorine, bromine or iodine. The aryl groups can be substituted on the ortho-, meta- or para-position on the aryl ring and two or more of the substituents can optionally form additional rings giving rise to cycloalkyl, heterocyclic alkyl, aromatic and heteroaromatic rings of 3-8 C-atoms. Preferably, the aryl groups are substituted with one or more methylene halide groups, more preferably this methylene halide group is a trifluoromethyl group.

Preferably R³ is a linear or branched alkyl group with 1-20 C-atoms which might or might not contain additional heteroatoms and/or functional groups. Two or more of the C-atoms can optionally form additional rings giving rise to cycloalkyl or heterocyclic alkyl rings of 3-8 C-atoms. Suitable additional heteroatoms are at least one from the group of O, S, N and P. Suitable functional groups can be chosen, for example, among C₁-C₆ haloalkyl, -F, -Cl, -Br, -CN, -NO₂, -CO₂H, -CONH₂, -SO₃H, - PO₃H₂ or C₁-C₁₂ alkyl esters or amides, phenyl esters, benzyl esters of the groups - CO₂H, -CONH₂, -SO₃H, -PO₃H₂. More preferably R³ is a linear or branched alkyl group with 1-8 C-atoms. Most preferably, R³ is a methyl group.

Suitable salts of the imine according to formula (I) include, but are not limited to those obtained by using inorganic or organic acids such as HCl, H₂SO₄, H₃PO₄, CH₃CO₂H, CF₃CO₂H, CCl₃CO₂H, C₆H₅CO₂H, CF₃SO₃H, C₆H₅SO₃H. More preferably, these are HCl, H₂SO₄, H₃PO₄, CH₃CO₂H salts; most preferably these are HCl salts. Salts of the imine according to formula (I) might result in an amine according to formula (II) with similar, different or no counterion when performing the process according to the invention.

The process of the invention is carried out in the presence of a transition metal catalyst, preferably an optically active transition metal catalyst, most preferably an optically active transition metal catalyst based on ruthenium, iridium, rhodium, palladium, iron or copper, more preferably ruthenium, iridium or rhodium. The optically active transition metal catalyst is preferably a homogeneous catalyst that is soluble in the reaction medium.

Suitable optically active transition metal catalysts are known to the person skilled in the art and are for example described in the Handbook of Homogeneous Hydrogenation (Ed. de Vries and Elsevier, Wiley, 2007, Chapters 23-34) and in Organic Reactions (Ed. Denmark et al., Wiley, 2009, Vol. 74, Chapter 1). These optically active transition metal catalysts are commercially available, prepared beforehand, or can be prepared in situ from any commercially available transition metal complex (also known as precursor) and an optically active ligand, or a combination of different ligands, of which one has to be optically active.

Non limiting examples of suitable metal precursors are bis(2-methylallyl)(1,5-cyclooctadiene)ruthenium, [RuCl₂(p-cymene)]₂, bis(1,5-cyclooctadiene)iridium tetrafluoroborate, bis(1,5-cyclooctadiene)diiridium dichloride, bis(cyclooctadiene)rhodium tetrafluoroborate, PdCl₂(CH₃CN)₂, Pd(CF₃CO₂)₂, Pd(CH₃CO₂)₂, FeCl₂, FeBr₂, Fe₃(CO)₁₂, Fe(CH₃CO₂)₂, [Fe(OH₂)₆](BF₄)₂, (NHEt₃)[Fe₃H(CO)_{11]}, CuCl, [CuH(Ph₃)]₆, Cu(NO₃)(PAr₃)₂ with Ar representing either a phenyl or a 3,5-xylyl group. In a preferred embodiment of the invention, the asymmetric hydrogenation process is performed in the presence of an iridium precursor. Most preferably, the metal precursor is bis(1,5-cyclooctadiene)diiridium dichloride, also depicted as [Ir(COD)Cl]₂.

The ligand in the process according to the invention can be any ligand suitable for asymmetric hydrogenation of imines. Optically active ligands and non optically active ligands are known to the person skilled in the art and are in general any compound which can donate electron density - ionically or by forming a covalent bond - to the relatively electron poor transition metal. Suitable non optically active ligands are for example dienes, arenes, carbenes, carboxylates, phosphines, N-sulfonated phosphinamines, phosphonates, phosphates, sulfonates, alcoholates, borates, pyridines, amines, nitriles, halides.

Suitable optically active ligands are molecules possessing one or more stereocenters, used in their enantiopure, racemic or enantioenriched form. An optically active ligand should be considered enantiopure (optically pure) when possessing stereocenters with at least 95% enantiomeric excess (ee). Suitable optically active ligands can be either monodentate, bidentate, tridentate or tetradentate. The above defined ligands may be characterized by the non limiting presence of one, more or combinations of cyclic or acyclic heteroatom-containing metal-binding functional groups such as phosphines, phosphites, phosphinates, phosphoramidites, sulfonamido-phosphoramidites, phosphinoxides, N-heterocyclic carbenes, diamines, imidazoles, aminoalcohols, diols, oxazolines, imines and amino acids (including the corresponding esters, carbamates and amides).

Preferably, the optically active ligand according to the process of the invention is a bidentate ligand containing one phosphine and one or more of the above mentioned metal binding moieties, with at least one chiral stereocenter and is especially an optically pure stereoisomer (enantiomer or diastereoisomer). Suitable but not limiting examples of such optically active bidentate ligands and related families if applicable, known to the person skilled in the art, are: BINAP, PhanePhos, ToIBINAP, Catasium, DuPhos, MeBICP, MeOBICP, BINAPHANE, SDP, DeGuPhos, JosiPhos, METAMORPhos, MeOMeOBiMEPH, DuanPhos, Me-f-KetalPhos, Fap, ChiraPhos, SegPhos, SkewPhos, dppach, TangPhos, MandyPhos, WalPhos, TaniaPhos, DIPAMP, NorPhos, BPE, POX, BiPhep, TunePhos, PHOX.

Preference is given to the so called P^{∧}N bidentate ligands which bind the metal center via a phosphine group and a nitrogen group. More preferably, the nitrogen group is an oxazoline. Most preferably, the phosphine and the oxazoline groups are bonded to different atoms of a carbon chain having 2-4 C-atoms; or are each bonded directly or indirectly via the cyclopentadienyl rings of a ferrocenyl moiety. Most preferred bidentate ligands are represented by ferrocene-based ligands according to formula (III) 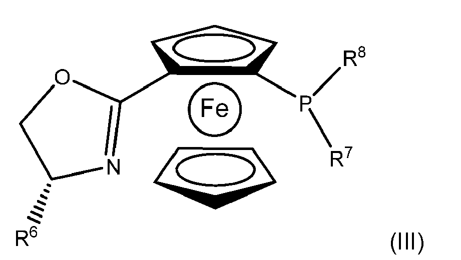 and its mirror image, wherein R⁶, R⁷ and R⁸ represent independently of each other an aryl, alkyl, aralkyl, alkenyl group with 1-10 C-atoms. More preferably, R⁶ is an alkyl group such as isopropyl, tert-butyl, or a phenyl group. Most preferably, R⁶ is an isopropyl group. Preferably R⁷ and R⁸ represent independently of each other a phenyl or a naphthyl group. Suitable substituents for the phenyl or naphthyl groups include, but are not limited to one or more hydrogen, alkyl, alkoxy (-OR⁹), carboxylate (-CO(O)R⁹), carbonyl (-C(O)R⁹), nitro (-NO₂), cyano (-CN), amino (-NR⁹R¹⁰) or methylene halide groups; wherein R⁹ and R¹⁰ represent independently from each other an aryl, alkyl, aralkyl, alkenyl group with 1-10 C-atoms; wherein the carbonyl groups also include but are not limited to ketones, aldehydes and amides, and wherein the methylene halide groups are of the formula ―CXₙH₃₋ₙ, wherein X can be fluorine, chlorine, bromine or iodine. The aryl groups can be substituted on the ortho-, meta- or para-position on the aryl ring. Most preferably, R⁷ and R³ are phenyl groups.

The asymmetric hydrogenation of the invention is performed in the presence of a nitrogen protecting reagent. Suitable nitrogen protecting reagents are known to the person skilled in the art and are, for example, described in the Protective Groups in Organic Synthesis (Ed. T. W. Greene, P.G.M. Wuts, Wiley, 1999, 3rd edition). Preferably, the nitrogen protecting reagent is a reagent according to formula (IV)

PG-Y (IV),

wherein Y is a halogen or a carboxylate (R¹¹CO₂-) residue and PG is an acyl (R¹²CO-), a phosphonyl (R¹³₂PO-), a sulfonyl (R¹⁴SO₂-) or a silyl (R¹⁵R¹⁶R¹⁷Si-) protecting group. Suitable R¹¹ residues include but are not limited to hydrogen, aryl, alkyl, haloalkyl, alkoxy or amino groups; most preferably R¹¹ is methyl, trifluoromethyl or phenyl. Suitable acyl protecting groups include but are not limited to those possessing R¹² being a hydrogen, aryl, alkyl, haloalkyl, alkoxy or amino groups; most preferably R¹² is methyl, trifluoromethyl or phenyl. Suitable phosphonyl protecting groups include but are not limited to those possessing R¹³ being a phenyl, benzyl, phenoxy or isopropoxy. Suitable sulfonyl protecting groups include but are not limited to those possessing R¹⁴ being a methyl, trifluoromethyl, phenyl or p-tolyl. Suitable silyl protecting groups include but are not limited to those possessing R¹⁵ , R¹⁶ and R¹⁷ each independently from each other being a chloro, methyl, phenyl or tert-butyl. More preferably, the nitrogen protecting group PG is an acyl group; more preferably Y is a carboxylate; most preferably the nitrogen protecting reagent is an anhydride and even more preferably acetic anhydride.

In an embodiment of the invention, the ratio between the nitrogen protecting reagent and the imine according to formula (I) is less than 10:1 mol equivalent, preferably less than 5:1 mol equivalent, more preferably less than 2:1 mol equivalent and most preferably less than 3:2 mol equivalent.

In another embodiment of the present invention, the process of the invention is performed in the absence or in the presence of solvents. Suitable solvents, which can be used alone or as a mixture of solvents, include, but are not limited to: protic solvents, such as methanol, ethanol, isopropanol, n-butanol and trifluoroethanol; aliphatic and aromatic hydrocarbons, such as n-pentane, n-hexane, n-heptane, cyclohexane, methylcyclohexane, benzene, toluene and xylene; ethers, such as diethyl ether, diethylene glycol dimethyl ether, methyl tert-butyl ether, 1,4-dioxane, tetrahydrofuran, 2-methyltetrahydrofuran; halogenated hydrocarbons, such as methylene chloride, chloroform, 1,1,2,2-tetrachloroethane and chlorobenzene; esters, lactones and anhydrides, such as ethyl acetate, butyrolactone, valerolactone and acetic anhydride; acid amides, nitriles and lactams, such as dimethyl formamide, N,N-dimethyl acetamide, N-methyl pyrrolidinone, 1,3-dimethyl-2-imidazolidinone and acetonitrile; ketones, such as acetone, dibutyl ketone, methyl isobutyl ketone and methoxy acetone; sulfoxides, such as 2,3,4,5 tetrahydrothiophene-1,1-dioxide, dimethyl sulfoxide and sulfolane. Preferably, the solvent is acetone, methyl tert-butyl ether, ethyl acetate, n-heptane and any mixture thereof. More preferably, the solvent is methyl tert-butyl ether.

In yet another embodiment of the invention, the substrate according to formula (I) to transition metal catalyst molar ratio is more than 500:1 mol equivalent, preferably more than 1000:1 mol equivalent, more preferably more than 5000:1 mol equivalent, most preferably more than 10000:1 mol equivalent.

The process according to the invention is carried out preferably at a temperature of from -20°C to 200°C, especially from 0°C to 60°C and more preferably from 20°C to 40°C.

Preferably, the process according to the invention is performed at a hydrogen pressure between 0.001 and 300 bar, preferably between 5 and 80 bar, more preferably between 20 and 40 bar.

The process according to the invention comprises the optional use of additives. An additive is a compound added to the reaction mixture to enhance the reaction rate, and/or increase the enantioselectivity. Additives are used preferably in amounts of from 0.1 to 10⁶ mol equivalents, more preferably from 0.5 to 1000 mol equivalents, based on the transition metal catalyst. Suitable additives are organic and inorganic compounds, among others salts, alcohols, bases or acids. Suitable but not limiting examples are ammonium salts, such as ammonium chloride, bromide and iodine, tetra-alkyl ammonium salts having 1-6 C-atoms on the alkyl residue, imidazolium salts, phenols, trifluoroethanol, pyridines, phtalimide, I₂, HCO₂H, CH₃CO₂H, CF₃CO₂H, C₆H₅CO₂H, CF₃SO₃H, p-tolyl-SO₃H, AgSbF₆, iPrOK, tert-BuOK, NaBH₄, AuCl₃, TiCl₄, HBF₄, C₆H₅B(OH)₂, C₆H₅PO₃H₂, BEt₃, NEt₃, CeCl₃, CuCl, InCl₃, Sc(OTf)₃, Yb(OTf)₃, BiCl₃ or FeCl₃.

The product of the hydrogenation process according to the invention is an optically active N-protected chiral amine of formula (II) and salts thereof. R¹, R² and R³ in formula (II) are the same as defined for R¹, R² and R³ formula (I). Salts of formula (II) will depend on the process and can be but are not limited to the corresponding HCl or CH₃CO₂H salt. These salts are not by definition the same as the salts of formula (I) and in another option such salts can be absent. Preferably, R¹ is a phenyl group; more preferably, R¹ is a substituted phenyl group; most preferably, R¹ is a phenyl group disubstituted on the meta-positions; even more preferably, R¹ is a phenyl group disubstituted on the meta-positions by trifluoromethyl groups. Preferably, R² and R³ are each, independently from each other, a linear or branched alkyl group of 1-8 C-atoms; more preferably R² and R³ are a methyl group. Furthermore, the product of the hydrogenation process according to the invention is an optically active N-protected chiral amine of formula (II), PG in formula (II) being a nitrogen protecting group introduced by the reaction of a nitrogen protecting reagent according to formula (IV) with the product of the asymmetric hydrogenation reaction. Preferably, PG is an acyl group; more preferably PG is an acetyl group.

In a further embodiment of the invention, the optically active N-protected amine product of the asymmetric hydrogenation can be isolated as such or can be deprotected, as also known to the person skilled in the art and described in the Protective Groups in Organic Synthesis (Ed. T. W. Greene, P.G.M. Wuts, Wiley, 1999, 3rd edition). In another preferred embodiment of the invention, the deprotection is done in situ. In a preferred embodiment of the invention, the asymmetric hydrogenation process of the invention and the deprotection are performed in the same pot. Suitable but not limiting deprotecting agents include acids such as HCl, HBr, HF, H₂SO₄, CH₃CO₂H, CF₃CO₂H and mixtures thereof. The resulting deprotected chiral amine product of the hydrogenation of the invention might be isolated as the corresponding salt of the acid used during the deprotection or can be optionally isolated as free amine by a reaction with a base, such as an inorganic base like NaOH or more preferred an aqueous solution of NaOH.

In one aspect of the invention, the obtained deprotected chiral free amine can be further reacted to obtain any pharmaceutically relevant derivative. Such reactions can be but are not limited to alkylation, acylation or reactions with an organic or inorganic acid to produce any pharmaceutically acceptable salt. Furthermore, the reactions can be combined in any sequence. In the context of the present invention, pharmaceutically acceptable salts are salts that are generally safe, non-toxic and neither biologically nor otherwise undesirable. Any of such derivatives possess the desired pharmacological activity of the parent compound.

The invention further relates to all possible combinations of different embodiments and/or preferred features according to the process according to the invention as described herein.

The invention will be elucidated with reference to the following examples, without however being restricted by these:

### EXAMPLES

### Example 1. Synthesis of N-(1-(3,5-bis(trifluoromethyl) phenyl)ethylidene)methanamine

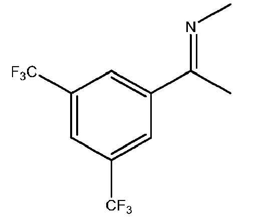

Under an atmosphere of N₂, 1-(3,5-bis(trifluoromethyl)-phenyl)ethanone (103.8 g, 0.406 mol) was dissolved in 2M CH₃NH₂ in THF (300 mL).

To this solution Ti(OiPr)₄ (69.0 g, 0.243 mol) was added and the mixture was allowed stirred for 2h at room temperature. An additional amount of 2M CH₃NH₂ in THF (25 mL) was subsequently added after 2h and 4h of reaction time. The mixture was concentrated under reduced pressure and the resulting crude mixture was added to heptane (200 mL). The TiO₂ was removed by filtration over Na₂SO₄. The reaction mixture was concentrated and the desired N-(1-(3,5-bis(trifluoromethyl) phenyl)ethylidene)methanamine was isolated by distillation under reduced pressure (77% yield).

### Example 2. Transition metal catalyst preparation:

[Ir(COD)Cl]₂ (4.2 mg, 6.25 µmol) and a bidentate ligand (13.1 µmol) or a monodentate ligand (26.2 mmol) were heated for 0.5 h in CH₂Cl₂ (0.5 mL) under N₂. The solution was cooled to room temperature and diluted with a 0.05M solution of I₂ in CH₂Cl₂ (0.5 mL). The catalyst stock solution was allowed to stir for 0.5 h at room temperature before being used.

### Example 3.

The following table contains non limiting examples that show the usefulness of the process described. The transition metal catalyst was prepared as described in Example 2. and used as such in the hydrogenation reactions. A standard hydrogenation protocol is further described in more detail in Example 4.

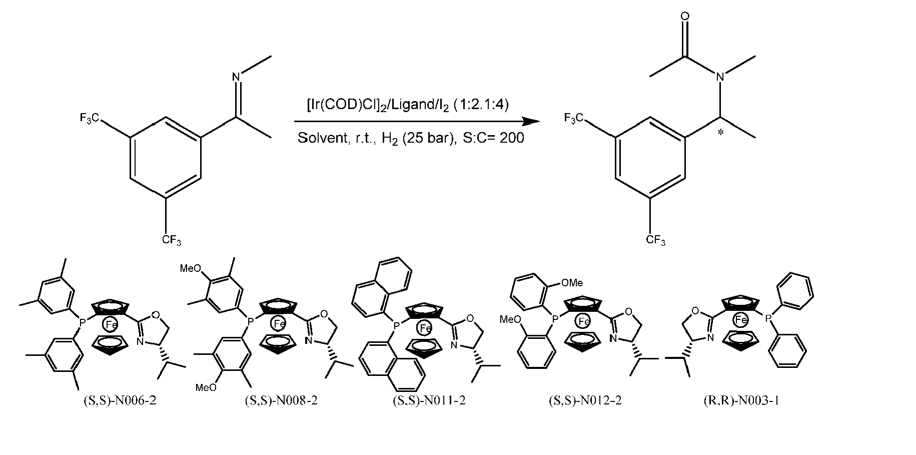

| Entry | Ligand | S/C | Ac₂O (eq.) | Solvent | Time (h) | Conversio (%) | Ee^{(a)} (%) |
|---|---|---|---|---|---|---|---|
| 1 | N003-1 | 200 | 1.1 | Acetone | 24 | 96 | -86 |
| 2 | N006-2 | 200 | 1.1 | Acetone | 24 | 96 | 95 |
| 3 | N008-2 | 200 | 1.1 | Acetone | 24 | 95 | 95 |
| 4 | N011-2 | 200 | 1.1 | Acetone | 24 | 74 | 66 |
| 5 | N012-2 | 200 | 1.1 | Acetone | 24 | 71 | 75 |
| 7 | N008-2 | 1000 | - | MTBE | 24 | 46 | 89 |
| 8 | N008-2 | 1000 | - | EtOAc | 24 | 22 | 90 |
| 9 | N008-2 | 1000 | - | Heptane | 24 | 3 | 92 |
| 10 | N008-2 | 1000 | - | Toluene | 24 | 14 | 91 |
| 11 | N008-2 | 1000 | 1.1 | MTBE | 24 | 94 | 93 |
| 12 | N008-2 | 1000 | 1.1 | EtOAc | 24 | 93 | 93 |
| 13 | N008-2 | 1000 | 1.1 | Heptane | 24 | 93 | 86 |
| 14 | N008-2 | 1000 | 1.1 | Toluene | 24 | 93 | 92 |
| 15 | N003-1 | 2000 | 1.1 | MTBE | 22 | 96 | -88 |
| 16 | N003-1 | 5000 | 1.1 | MTBE | 22 | 96 | -89 |
| 17 | N003-1 | 10000 | 1.1 | MTBE | 22 | 96 | -90 |
| 18 | N003-1 | 20000 | 1.1 | MTBE | 40 | 96 | -90 |
| 19 | N003-1 | 25000 | 1.1 | MTBE | 40 | 96 | -91 |
| 20 | N003-1 | 50000 | 1.1 | MTBE | 40 | 96 | -91 |

| | | | | | | | |
|---|---|---|---|---|---|---|---|
| (a) Respectively, the (R)-enantiomer of the product was obtained when using the (R,R)-ligand and the (S)-enantiomer of the product was obtained when using the (S,S)-ligand | | | | | | | |

Conversion of N-(1-(3,5-bis(trifluoromethyl) phenyl)ethylidene)-methanamine to (R)-N-(1-(3,5-bis(trifluoromethyl)phenyl)ethyl)-N-methylacetamide was determined by GC; column: CPSil5CB (25m x 0.32mm x 1.2µm); temperature: 110°C (hold 3min) to 250°C (20°C/min, hold 1min).

Enantiomeric of (R)-N-(1-(3,5-bis(trifluoromethyl)phenyl)ethyl)-N-methylacetamide excess was measured by GC; column: GTA (50m x 0.32mm x 1.2µm); temperature 120°C (hold 11min) to 180°C (10°C/min).

### Example 4. Hydrogenation of N-(1-(3,5-bis(trifluoromethyl) phenyl)ethylidene)methanamine to obtain (R)-N-(1-(3,5-bis(trifluoromethyl)phenyl)ethyl)-N-methylacetamide:

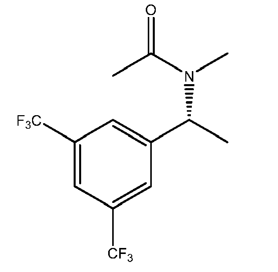

A reactor was charged with a solution of N-(1-(3,5-bis(trifluoromethyl) phenyl)ethylidene)methanamine (15.1 g, 56.2 mmol) and acetic anhydride (6.3 g, 62.0 mmol) in MTBE (75 mL). The complex stock solution (5.6 µmol) was added dropwise. The reactor was pressured to 25 bar with H₂ and the reaction mixture was stirred at room temperature for 23 hours. After which time, the reaction mixture was extracted with sat. NaHCO₃ (sat., 50 mL) and water (50 mL). The organic layer was concentrated under reduced pressure and the crude product (R)-1-(3,5-bis(trifluoromethyl)phenyl)-N-methylethanamine was obtained as a viscous oil (24.9 g, 90% ee), which was dissolved in heptane (110 mL) and upon cooling to 0°C precipitated from the solution. The precipitate was then filtrated and dried. Subsequent crystallization yielded further enriched (R)-N-(1-(3,5-bis(trifluoromethyl)phenyl)ethyl)-N-methylacetamide (99.5% ee).

### Example 5.Deprotection and isolation of (R)-N-(1-(3,5-bis(trifluoromethyl)phenyl)ethyl)-N-methylacetamide to obtain (R)-1-(3,5-bis(trifluoromethyl)phenyl)-N-methylethanamine

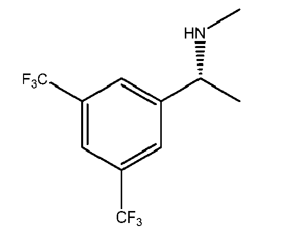

The reaction vessel was charged with (R)-N-(1-(3,5-bis(trifluoromethyl)phenyl)ethyl)-N-methylacetamide (17.3 g, 55.1 mmol, >99.5% ee), propanol (65 mL) and HCl (6N aq., 65 mL). The mixture was heated to reflux (95°C) for 20h whilst distilling an azeotropic mixture. The desired product was obtained, after concentration, as a slurry of the corresponding HCl salt. The slurry was diluted with water (120 mL) and the pH of the heterogeneous water layer was adjusted to pH 12 by slow addition of NaOH (32 w/w % in water) in the presence of CH₂Cl₂ (50 mL) whilst maintaining the temperature at 20°C.

The free (R)-1-(3,5-bis(trifluoromethyl)phenyl)-N-methylethanamine was then extracted into the organic phase. The CH₂Cl₂ layer was separated and the water layer extracted twice with CH₂Cl₂ (2 x 50 mL). The combined organic layers were dried over anhydrous Na₂SO₄, filtrated and concentrated under reduced pressure yielding crude the crude amine (94% yield), which was purified by distillation under reduced pressure (10.7 g, 99.6% ee).

## Claims

1. Process for the asymmetric hydrogenation of imines according to formula (I) 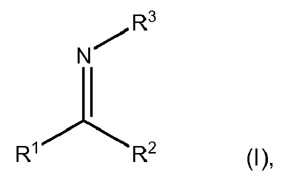 and salts thereof, wherein R¹ and R² are each independently any alkyl, aryl, aralkyl, alkenyl or alkynyl group with 1-20 C-atoms and R³ is hydrogen atom, an alkyl, aralkyl, alkenyl or alkynyl group with 1-20 C-atoms and wherein R¹, R² and R³ can optionally form a ring together with the atoms to which they are bound, it being possible for R¹, R² and R³ to also contain one or more heteroatoms and substituents, in the presence of a transition metal catalyst, one or more ligands, a nitrogen protecting reagent and optionally an additive, wherein a product of formula (II) and salts thereof 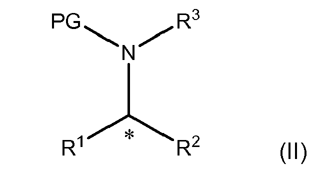 are formed, wherein PG stands for a protecting group introduced by the reaction of the nitrogen protecting reagent with the product of the asymmetric hydrogenation reaction.

2. Process according to claim 1, wherein the transition metal catalyst is based on iridium, ruthenium, rhodium, iron, palladium or copper.

3. Process according to claim 2, wherein the transition metal catalyst is based on [Ir(COD)Cl]₂.

4. Process according to any of claims 1 to 3, wherein the ligand is a monodentate phosphoramidite, a diphosphine or a P^{∧}N ligand.

5. Process according to claim 4, wherein the P^{∧}N ligand is a ferrocene-based ligand.

6. Process according to any claims 1 to 5, wherein the nitrogen protecting reagent is a reagent of formula (IV)
PG-Y (IV),
wherein Y is a halogen or a carboxylate residue and PG is an acyl, a phosponyl, a sulfonyl or a silyl protecting group.

7. Process according to claim 6, wherein the nitrogen protecting reagent is an anhydride.

8. Process according to any of claims 1 to 7, wherein the ratio between the nitrogen protecting reagent and the imine according to formula (I) is less than 2:1 mol equivalent.

9. Process according to any of claims 1 to 8, wherein a solvent is present and wherein the solvent is acetone, methyl tert-butyl ether, ethyl acetate or n-heptane.

10. Process according to any of claims 1 to 9, wherein the ratio between the imine according to formula (I) and the catalyst is more than 5000:1 mol equivalent.

11. Process according to any of claims 1 to 10, wherein R³ in formula (I) is an alkyl with 1-8 C-atoms.

12. Process according to any of claims 1 to 11, wherein the salts of the imine according to formula (I) are those obtained by reaction with acetic acid, hydrochloric acid, phosphoric acid or sulfuric acid.

13. Process according to any of claims 1 to 12, wherein the resulting protected chiral amine according to formula (II) is deprotected.

14. Process according to claim 13, wherein the deprotection process is performed in situ.

15. Process according to any of claims 13 to 14, wherein the deprotected chiral amine is further reacted with an acid to produce any pharmaceutically acceptable salt.

16. Process according to any of claims 13 to 15, wherein the deprotected chiral amine is further subjected to an acylation or alkylation reaction.

17. Chiral amine obtained from the process according to any of claims 1 to 12, wherein the product of the asymmetric hydrogenation of the invention is an N-protected amine according to formula (II) wherein R¹ is a phenyl group disubstituted on both meta-positions by trifluoromethyl groups, wherein R² and R³ are methyl groups and wherein PG is an acetyl group.
